# EUROPEAN PATENT APPLICATION

(11) **EP 4 509 582 A1**
(43) Date of publication of application: **19.02.2025**
(21) Application number: 22740424.1
(22) Date of filing: 13.04.2022
(51) Int. Cl.: C10G 2/00, C07C 1/04, C07C 29/151, C10B 53/07, C10J 3/66, C10K 1/08, C10K 3/02

(54) **METHOD AND SYSTEM FOR MATERIAL VALORIZATION OF WASTE FOR THE CO-PRODUCTION OF HYDROGEN AND HYDROCARBONS OR ALCOHOLS**

(71) Applicant: IET Ecology S.L., 01013 Vitoria-Gasteiz, Alava (ES)
(72) Inventor: OBREGÓN BENGOA, Iker, 01013 Vitoria-Gasteiz, Alava (ES); RUIZ DE ANGULO GABILONDO, Juan, 01013 Vitoria-Gasteiz, Alava (ES); CAUBILLA ANGULO, Juan Manuel, 01013 Vitoria-Gasteiz, Alava (ES)
(74) Representative: Pons
(86) International application number: PCT/ES2022/070231
(87) International publication number: WO 2023/198940

(57) **Abstract**

The present invention relates to the material valorization of waste through thermo-chemical treatments, in order to obtain products with high added value, such as hydrogen and hydrocarbons or alcohols, and the systems to carry them out.

## Description

### OBJECT OF THE INVENTION

The present invention relates to the material valorization of waste through thermo-chemical treatments, in order to obtain products with high added value.

### BACKGROUND OF THE INVENTION

Currently, a large amount of waste generated by human activity is deposited in landfills. The socio-economic and environmental damage they generate makes it desirable to develop waste valorization technologies in order to reduce the amount of waste that does not have a useful purpose.

The recovery route can be either energetic, through the incineration of waste to obtain electrical power, or material, in which the waste is transformed into different chemical compounds such as hydrogen, hydrocarbons or alcohols, among others.

An example of energy recovery is described in the patent under publication number EP2318157 wherein a system and method for the pyrolytic processing of municipal and domestic waste is proposed. The waste is sequentially passed through sorting, grinding, drying and accumulating units, and sent to a pyrolysis reactor for its pyrolytic treatment. The synthesis gas produced in pyrolysis undergoes a series of cleaning steps and is subsequently used as a working medium for an electrical power generation unit. Carbon dioxide is also obtained in the process as a result of the synthesis gas purification processes. The objective of this recovery is to obtain energy and carbon dioxide.

The patent under publication number US10280377 describes a system comprising: a pyrolysis chamber and a vapor cracking unit, in order to obtain a synthesis gas that is used in the cogeneration of energy and/or steam.

In the documents of the state of the art, the valorization of waste is both energetic and material. In the present invention, a method has been developed in which valorization is exclusively material, producing hydrogen, hydrogen and hydrocarbons, or hydrogen and alcohols, with maximum efficiencies in relation to the products obtained.

### DESCRIPTION OF THE INVENTION

Due to the growing concern for the environment and increasingly strict regulations, the efficient management of urban, industrial and agricultural solid waste, among others, is an increasingly important problem.

The objective of the valorization is the transformation of the waste materials into high value-added products or into raw materials for other uses and processes, with the consequent environmental and economic benefit arising from the prevention of their deposition in landfills.

The present invention describes a method and a system, based on a thermal treatment of waste, which generates different products with high added value such as hydrogen, hydrocarbons or alcohols. Additionally, hydrogen production is enhanced through the integration of the process streams and when it is co-produced with hydrocarbons or alcohols.

The method of the invention comprises a pyrolysis reaction where the starting waste is decomposed by external heating of the reactor and under a reducing atmosphere. From the products obtained in the pyrolysis, the obtained solid phase can optionally be separated. After the pyrolysis, the method comprises a step of cracking the pyrolysis products, in which they react in the presence of oxygen and steam to obtain a synthesis gas. At the outlet of the cracking step, the obtained raw synthesis gas is cooled and purified.

A fraction of this gas is mixed with steam and it reacts, in the presence of a catalyst, to obtain a hydrogen-enriched synthesis gas. A fraction of this hydrogen-enriched gas is treated to extract hydrogen as a product, while the remainder is mixed with the previously unused fraction of purified synthesis gas and it reacts, in the presence of a second catalyst, to obtain a second product derived from the synthesis gas.

This method, in which hydrogen and a second product (hydrocarbons or alcohols) are co-produced, generates a synergy in the integration of the process that allows better productivities to be obtained compared to the process oriented to hydrogen production as the sole product. In this way, starting from the same amount of raw material, greater amounts of hydrogen are obtained through its co-production than when hydrogen is the only product, in addition to significant amounts of a second product with high added value.

Therefore, a first aspect of the invention relates to a waste valorization method for the co-production of hydrogen and hydrocarbons or alcohols that comprises the steps of:
a) subjecting to pyrolysis the starting waste, by means of external heating and under a reducing atmosphere, to obtain pyrolysis products;
b) optionally, separating the solid fraction from the pyrolysis products;
c) cracking the products obtained in the pyrolysis step a), or in step b) after separating the solid fraction, in the presence of oxygen and a first stream of steam, to obtain raw synthesis gas;
d) cooling the raw synthesis gas obtained in c);
e) purifying the raw synthesis gas obtained in d) to obtain purified synthesis gas;
f) conversion of the mixture of a first preheated fraction of the gas obtained in e), with a second steam stream, in the presence of a catalyst, to obtain a hydrogen-enriched synthesis gas;
g) separating hydrogen from a first fraction of the hydrogen-enriched synthesis gas, to obtain hydrogen and a tail gas from hydrogen separation;
h) synthesis of a product selected from hydrocarbons or alcohols, from a preheated mixture of a second gas fraction obtained in e), with a second fraction of the hydrogen-enriched synthesis gas obtained in f);
i) fractionation and/or purification of the product obtained in h), obtaining different streams of hydrocarbons or alcohols, a stream of process water and a stream of process tail gas,
where the steps from f) to g) and h) to i) are parallel in time.

The present invention also relates to the method for the production of hydrogen in which the different process streams are integrated to obtain hydrogen as the only product. The method comprises the same steps a) to g) described in the previous paragraph. To improve the hydrogen production efficiency, the external heating in step a) is carried out by combustion of a stream selected from a fraction of the tail gas from step g) and/or a fraction of the purified synthesis gas from step e); mixed with a preheated air stream, which generates a flue gas stream, which releases heat for the external heating of step a) and, after the pyrolysis step, releases heat to a stream and/or fraction selected from: a water stream to generate steam necessary for steps c) and/or f) and/or an air stream to obtain the preheated air stream and/or the purified synthesis gas fraction processed in step f). Therefore, the invention relates to the method for material valorization of waste for the production of hydrogen that comprises the steps of:
a) subjecting to pyrolysis the starting waste, by means of external heating and under a reducing atmosphere to obtain pyrolysis products;
b) optionally, separating the solid fraction from the pyrolysis products;
c) cracking the products obtained in step a) of pyrolysis, or in step b) after separating the solid fraction, in the presence of oxygen and a first stream of steam, to obtain raw synthesis gas;
d) cooling the raw synthesis gas obtained in c);
e) purifying the raw synthesis gas obtained in d) to obtain purified synthesis gas;
f) converting a mixture of the first preheated fraction of the gas obtained in e), with a second steam stream, in the presence of a catalyst, to obtain a hydrogen-enriched synthesis gas;
g) separation of the hydrogen-enriched synthesis gas, to obtain hydrogen and a tail gas from hydrogen separation;
furthermore, it comprises a step of combustion of a fuel stream selected from a fraction of the tail gas from step g) and/or a fraction of the purified synthesis gas from step e); mixed with a preheated air stream, which generates a flue gas stream, which releases heat to the external heating of step a) and, after the pyrolysis step, releases heat to at least one stream and/or fraction selected from: a stream of water to generate the steam necessary for steps c) and f) and/or an air stream to obtain the preheated air stream and/or the first fraction of the gas obtained in e), preheated in f).

The term "material valorization" relates to the use of a waste to obtain a material product that can be used in a socio-economic activity.

The term "waste" in the present description relates to a material, discarded from other processes, capable of undergoing chemical decomposition by heating producing, at least, a combustible gas. Examples of waste are, among others: solid urban waste, waste from industrial activities or biomass waste.

The term "pyrolysis" relates to thermolysis under a reducing atmosphere.

The term "external heating" relates to indirect heating, that is, supplying heat externally by electrical heating, external combustion or other means.

The term "cracking" in the present invention relate to the thermo-chemical conversion of tars into lower molecular weight molecules, optionally in the presence of a catalyst.

The term "tars" in the present invention relates to the organic compounds, with a molecular weight higher than that of methane, coming from the pyrolysis reactor.

The term "synthesis gas" relates to a gas comprising primarily hydrogen and carbon monoxide, and which may contain smaller amounts of carbon dioxide, methane, water, nitrogen and tars.

The term "hydrocarbon" includes straight or branched chain compounds of the alkane or alkene type with a carbon number from 5 to 50.

The term "alcohols" relates to straight or branched chain mono-alcohols with a carbon number from 1 to 5.

Another aspect of the invention is the system for carrying out the first method of the invention, the system for the co-production of hydrogen and hydrocarbons or alcohols, comprising at least:
a pyrolysis unit,
a cracking unit,
a purification unit,
a synthesis gas conversion unit,
a hydrogen separation unit,
a hydrocarbon or alcohol synthesis unit and
a fractionation and/or purification unit for hydrocarbons or alcohols.

Finally, the invention relates to a system for the production of hydrogen comprising, at least:
a pyrolysis unit,
a cracking unit,
a purification unit,
a synthesis gas conversion unit,
a hydrogen separation unit and
a unit for the recovery of residual heat from the flue gas.

The cracking unit in both systems comprises a cracking reactor and a cooling unit for the raw synthesis gas obtained.

### DESCRIPTION OF DRAWINGS

To complement the description that is being made, and for a better understanding of the features of the invention, according to a preferred example of its practical embodiment, a set of drawings is attached as an integral part of said description, wherein, with an illustrative and non-limiting nature, the following has been represented:

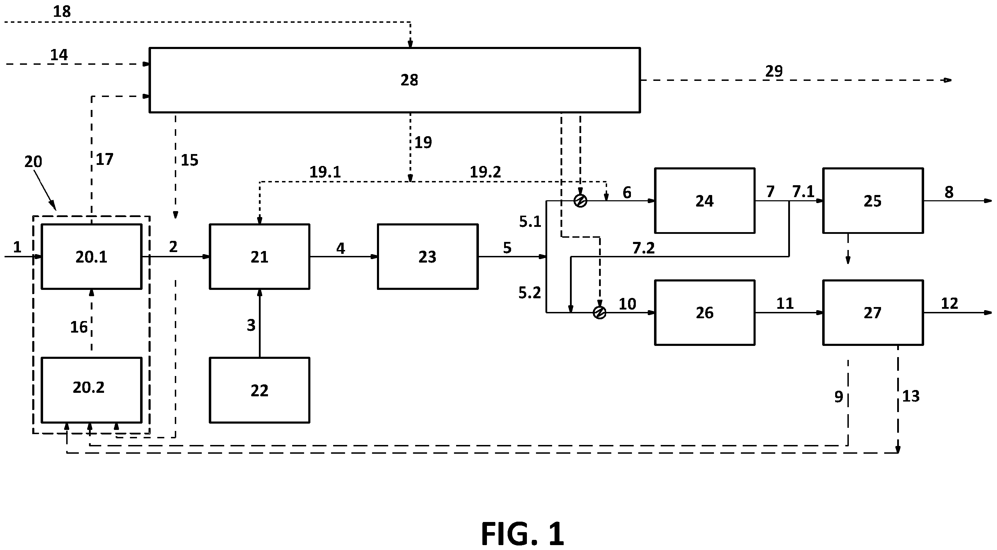

Figure 1 shows a scheme of an embodiment of the invention referred to the co-production of hydrogen and hydrocarbons or alcohols.

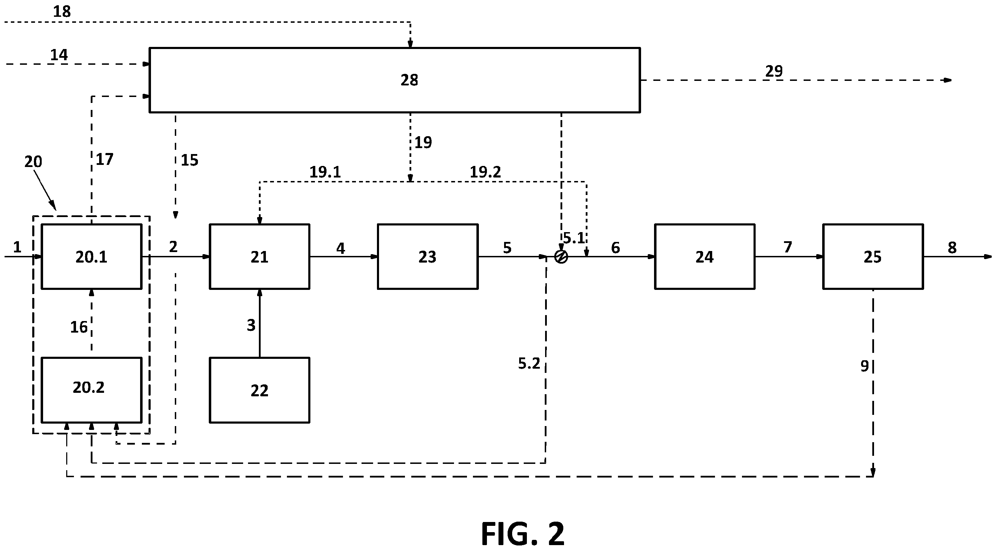

Figure 2 shows a scheme of an embodiment of the invention referred to the production of hydrogen.

### PREFERRED EMBODIMENT OF THE INVENTION

As indicated in the first aspect of the invention, it relates to a waste valorization method for the co-production of hydrogen and hydrocarbons or alcohols that comprises the steps of:
a) subjecting to pyrolysis the starting waste (1), by means of external heating and under a reducing atmosphere, to obtain pyrolysis products (2);
b) optionally, separating the solid fraction from the pyrolysis products;
c) cracking the products obtained in the pyrolysis step a), or in step b) after separating the solid fraction, in the presence of oxygen (3) and a first steam stream (19.1), to obtain raw synthesis gas (4);
d) cooling the raw synthesis gas (4) obtained in c);
e) purification of the raw synthesis gas (4) obtained in d) to obtain purified synthesis gas (5);
f) conversion of a mixture (6) of a first preheated fraction of the gas (5.1) obtained in e), with a second steam stream (19.2), in the presence of a catalyst, to obtain a hydrogen-enriched synthesis gas (7);
g) separating hydrogen from a first fraction of the hydrogen-enriched synthesis gas (7.1), to obtain hydrogen (8) and a tail gas (9) from the separation of hydrogen;
h) synthesis of a product (11) selected from hydrocarbons or alcohols, from the preheated mixture (10), of a second fraction of gas obtained in e) (5.2), with a second and preheated fraction of the hydrogen-enriched synthesis gas obtained in f) (7.2);
i) fractionation and/or purification of the product obtained in h), obtaining different streams (12) of hydrocarbons or alcohols, a stream of process water and a stream of tail gas from the process (13),
wherein steps f) to g) and h) to i) are parallel in time.

Preferably, the method of the invention integrates the heating and cooling energy needs of the different process streams so that the system can operate without the need for external thermal energy, for example, fossil fuels. The indirect heating of the pyrolysis step a) allows to obtain a high quality pyrolysis gas (without dilution with inert compounds) while it makes it possible to use the gas generated for heating the pyrolyzer, flue gas (16) and (17), for heating the rest of the process streams, maximizing its efficiency.

Therefore, preferably, the method described above has a step of combustion of a fuel stream selected from a fraction of tail gas (9) from step g) and/or a fraction of tail gas (13) from step i), together with a preheated air stream (15), which generates a flue gas stream (16), which gives off heat in the external heating of step a) and, after the pyrolysis step, the flue gas stream (17) from the pyrolyzer outlet, gives off heat, at least to a stream and/or fraction selected from: a stream of water (18) to generate steam (19) necessary for steps c) and f) and a stream of air (14) to obtain the preheated air stream (15) and/or the first fraction of the gas (5.1) from step f) and/or the mixture from step h) to preheat them. In another possible embodiment, the streams of the process itself are not used to generate the energy necessary for the pyrolysis, as detailed in the previous paragraph, and the flue gas stream (16) gives off heat, at least to one stream and/or fraction selected from: a stream of water (18) to generate steam (19) necessary for steps c) and f) and/or an air stream (14) to obtain the preheated air stream (15) and/or the first fraction of the gas (5.1) from step f) and/or the mixture from step h) to preheat them. From the heat release steps, a flue gas stream from heat exchange (29) is obtained.

Preferably in step h) hydrocarbons are synthesized by means of the Fischer-Tropsch reaction and in step i) the hydrocarbons are fractionated into the streams of desired products, optionally naphtha, distillates and paraffinic waxes.

Preferably, the cracking step c) is carried out in the presence of a catalyst.

Preferably in step h) alcohols are synthesized, more preferably methanol.

Preferably, a fraction or all the process water stream from step i) is recycled together with the feed water (18).

In one aspect of the invention, the different streams are integrated to produce hydrogen as the only product. In this embodiment, the waste valorization method for the production of hydrogen comprises the steps of:
a) subjecting to pyrolysis the starting waste (1), by means of external heating and under a reducing atmosphere, to obtain pyrolysis products (2);
b) optionally, separating the solid fraction from the pyrolysis products;
c) cracking the products obtained in the pyrolysis step a), or in step b) after separating the solid fraction, in the presence of oxygen (3) and a first steam stream (19.1), to obtain raw synthesis gas (4);
d) cooling the raw synthesis gas (4) obtained in c);
e) purification of the raw synthesis gas (4) obtained in d) to obtain purified synthesis gas (5);
f) conversion of a mixture (6) of a first preheated fraction of the gas (5.1) obtained in e), with a second steam stream (19.2), in the presence of a catalyst, to obtain a hydrogen-enriched synthesis gas (7);
g) separating the hydrogen-enriched synthesis gas (7), to obtain hydrogen (8) and a tail gas (9) from the separation of hydrogen;
it also comprises a step of combustion of a fuel stream selected from a fraction of the tail gas (9) from step g) and/or a fraction of the purified synthesis gas (5.2); mixed with a preheated air stream (15), which generates a flue gas stream (16), which gives off heat in the external heating of step a) and, after the pyrolysis step, gives off heat to at least one stream and/or fraction selected from: a stream of water (18) to generate steam (19) necessary for steps c) and f) and/or an air stream (14) to obtain the preheated air stream (15) and/or the fraction (5.1) prior to step f).

Both in the method for the co-production of hydrogen and hydrocarbons or alcohols, and in the method for the production of hydrogen, the following preferred embodiments are considered.

Preferably, the starting waste (1) is selected from: solid urban waste, waste generated by commercial and industrial activities and/or biomass, among other possible ones.

The starting waste (1) is preferably treated before step a) to adapt it to the needs of the method. More preferably, the starting waste (1) is selected by size through a sieve and/or crushed and/or pressed and/or dried. In particular, the particle size of the starting waste shall be less than 10 mm in the largest of its dimensions.

Preferably, in step b) the solid fraction is separated from the pyrolysis products. This product can be used as fuel or can be used to recover the metals present in this fraction.

An advantage of the present invention is the separation in steps of the thermo-chemical treatment of the waste, together with the optimization of the means to obtain the temperatures necessary for the different conversion reactions. In this way, a synthesis gas with minimal amounts of impurities and inert materials is obtained, in a system that minimizes thermal needs at high temperatures and, thus, results in high efficiencies.

Preferably, the pyrolysis step a) is carried out in a temperature range between 400°C and 650°C and the temperature of the cracking step c) is carried out at a temperature between 800°C and 1300°C to maximize the content of hydrogen and carbon monoxide in the synthesis gas, and minimize the tar content.

Preferably, the cooling step d) is carried out using the heat of the raw synthesis gas (4) to generate steam. Preferably the temperature of the cooled raw synthesis gas is between 250°C and 350°C.

This raw synthesis gas (4) can have different contaminants, both solid and gaseous, and preferably the purification requires one or more steps selected from: separation of solids, absorption in liquids and/or adsorption on solids.

Another advantage of the two methods, both the co-production of hydrogen and hydrocarbons or alcohols, as well as the hydrogen production method is the recovery and use in the same method of the water streams condensed in the process.

Thus, in a preferred embodiment, after conversion step f), the fraction of steam that has not reacted is condensed from the hydrogen-enriched gas (7), and a fraction of the condensed water is recycled together with the feed water (18).

In another preferred embodiment, after conversion step f), the hydrogen-enriched synthesis gas (7) is cooled by a stream of water that is subsequently heated to form steam (19.1) from the cracking step and/or the steam (19.2) from the conversion step.

As it has been said, the system for carrying out the first method of the invention, the system for the co-production of hydrogen and hydrocarbons or alcohols, comprises at least:
a pyrolysis unit (20),
a cracking unit (21),
a purification unit (23),
a synthesis gas conversion unit (24),
a hydrogen separation unit (25),
a hydrocarbon or alcohol synthesis unit (26) and
a unit for fractionation and/or purification of hydrocarbons or alcohols (27).

Preferably, the systems comprise an oxygen generation unit (22) that generates the oxygen for the cracking step. Preferably, the pyrolysis unit comprises a pyrolyzer (20.1) and at least one burner (20.2) to generate the heat necessary for pyrolysis.

In addition, preferably, the system for carrying out the waste recovery method for the production of hydrogen and hydrocarbons or alcohols comprises a section for recovering residual heat from flue gases (28). More preferably, the section for the recovery of residual heat from flue gases (28) comprises a burner.

### Example 1. Example of hydrogen production

The crushed urban solid waste was fed through a hopper, with a bulky material rejection mesh, from where it was dosed into the pyrolyzer, avoiding the entry of air into the reactor. In the pyrolyzer, the waste was subjected to a temperature of 450°C under a reducing atmosphere for its partial decomposition. The pyrolysis products then entered the cracking reactor where they were thermally converted into synthesis gas at 1200°C, in the presence of steam and oxygen. The raw synthesis gas was cooled to 330°C. The cooled raw synthesis gas then passed to a purification unit to remove contaminants, consisting of an absorption unit and an adsorption unit. A fraction of the purified synthesis gas was directed to the burners to supply heat to the pyrolyzer, while the rest was preheated in the unit for recovering residual heat from the flue gas and mixed with steam before being fed to the synthesis gas conversion reactor, wherein the CO reacts with steam to produce H₂ and CO₂ over a catalytic bed. The hydrogen-enriched synthesis gas was fed to a hydrogen separation unit by pressure swing adsorption (PSA) from which H₂ and a tail gas stream, that was used to supply heat to the pyrolyzer, were obtained.

In this method, 133 kg of hydrogen were obtained per ton of waste.

### Example 2. Example of co-production of hydrogen and hydrocarbons

The steps of pyrolyzer feed, pyrolysis, thermal conversion, raw synthesis gas cooling, and synthesis gas purification were performed in the same manner as in Example 1. As in Example 1, a fraction of the purified synthesis gas was preheated in the unit for the recovery of residual heat form the flue gas and mixed with steam before being introduced to the synthesis gas conversion reactor, wherein hydrogen-enriched synthesis gas was generated. A fraction of this stream was fed to a purification unit wherein hydrogen was obtained.

A mixture of a second fraction of purified synthesis gas and a fraction of hydrogen-enriched synthesis gas, preheated in the heat recovery unit, was directed to the hydrocarbon synthesis unit and the products thereof were separated into final product streams.

In this method, 144 kg of hydrogen and 64 kg of hydrocarbon were obtained per ton of waste.

### Example 3. Example of co-production of hydrogen and methanol

Example 3 was performed in the same manner as Example 2, except that the mixture of the second fraction of purified synthesis gas with a fraction of hydrogen-enriched synthesis gas was directed to a methanol synthesis unit, and the product thereof, was purified to obtain the final product.

In this method, 148 kg of hydrogen and 121 kg of methanol were obtained per ton of waste.

The examples of the invention demonstrate that, starting from the same amount and composition of waste, more hydrogen is obtained per ton of waste when it is co-produced with hydrocarbons or alcohols, methanol as indicated in Example 3.

## Claims

1. Method for the material valorization of waste for the co-production of hydrogen and hydrocarbons or alcohols comprising the steps of:
a) subjecting to pyrolysis the starting waste (1), by means of external heating and under a reducing atmosphere, to obtain pyrolysis products (2);
b) optionally, separating the solid fraction from the pyrolysis products;
c) cracking the products obtained in the pyrolysis step a), or in step b) after separating the solid fraction, in the presence of oxygen (3) and a first steam stream (19.1), to obtain raw synthesis gas (4);
d) cooling the raw synthesis gas (4) obtained in c);
e) purification of the raw synthesis gas (4) obtained in d) to obtain purified synthesis gas (5);
f) converting a mixture (6) of a first preheated fraction of the gas (5.1) obtained in e), with a second steam stream (19.2), in the presence of a catalyst, to obtain a hydrogen-enriched synthesis gas (7);
g) separating hydrogen from a first fraction of the hydrogen-enriched synthesis gas (7.1), to obtain hydrogen (8) and a tail gas (9) from the separation of hydrogen;
h) synthesis of a product (11) selected from hydrocarbons or alcohols, from the preheated mixture (10), of a second fraction of gas obtained in e) (5.2), with a second and fraction of the hydrogen-enriched synthesis gas obtained in f) (7.2);
i) fractionation and/or purification of the product obtained in h), obtaining different streams (12) of hydrocarbons or alcohols, a stream of process water and a stream of tail gas from the process (13),
wherein steps f) to g) and h) to i) are parallel in time.

2. Method according to claim 1, **characterized in that** it comprises a step of combustion of a fuel stream selected from a fraction of the tail gas (9) from step g) and/or a fraction of the tail gas (13) from step i), together with a preheated air stream (15), which generates a flue gas stream (16), which gives off heat in the external heating of step a) and, after the pyrolysis step, gives off heat, at least to one stream and/or fraction selected from: a stream of water (18) to generate steam (19) necessary for steps c) and f) and/or an air stream (14) to obtain the preheated air stream (15) and/or the first fraction of the gas (5.1) of step f) and/or the mixture of step h) to preheat them.

3. Method according to claim 1, **characterized by** a step of combustion of a fuel stream selected from a fraction of tail gas (9) from step g) and/or a fraction of tail gas (13) from step i), mixed with a preheated air stream (15), which generates a flue gas stream (16), which gives off heat to at least one stream and/or fraction selected from: a stream of water (18) to generate steam (19) necessary for the steps c) and f) and/or an air stream (14) to obtain the preheated air stream (15) and/or the first gas fraction (5.1) of step f) and/or the mixture of step h) to preheat them.

4. Method according to any one of the claims 1 to 3 **characterized in that** after conversion step f), the hydrogen-enriched synthesis gas (7) is cooled by a stream of water which is subsequently heated to form steam (19.1) for the cracking step and/or steam (19.2) for the conversion step.

5. Method according to any one of the claims 1 to 4 **characterized in that** after conversion step f), the unreacted steam fraction of the hydrogen-enriched gas (7) is condensed, and a fraction of the condensed water is recycled together with the feed water (18).

6. Method according to any one of the claims 1 to 5 **characterized in that** a fraction or all of the process water stream from step i) is recycled together with the feed water (18).

7. Method according to any one of the claims 1 to 6 **characterized in that** the pyrolysis step a) is carried out in a temperature range between 400°C and 650°C and the temperature of the cracking step c) is in a range between 800°C and 1300°C.

8. Method according to any one of the claims 1 to 7, **characterized in that** in step c), cracking is carried out in the presence of a catalyst.

9. Method according to any one of the claims 1 to 8, **characterized in that** in step h) methanol is synthesized.

10. Method according to any one of the claims 1 to 9, **characterized in that** the purification step e) comprises at least one step selected from: separation of solids, absorption in liquids and/or adsorption on solids.

11. Method for material valorization of waste for the production of hydrogen that includes the steps of:
a) subjecting to pyrolysis the starting waste (1), by means of external heating and under a reducing atmosphere, to obtain pyrolysis products (2);
b) optionally, separating the solid fraction from the pyrolysis products;
c) cracking the products obtained in the pyrolysis step a), or in step b) after separating the solid fraction, in the presence of oxygen (3) and a first steam stream (19.1), to obtain raw synthesis gas (4);
d) cooling the raw synthesis gas (4) obtained in c);
e) purification of the raw synthesis gas (4) obtained in d) to obtain purified synthesis gas (5);
f) converting a mixture (6) of a first preheated fraction of the gas (5.1) obtained in e), with a second steam stream (19.2), in the presence of a catalyst, to obtain a hydrogen-enriched synthesis gas (7);
g) separating the hydrogen-enriched synthesis gas (7), to obtain hydrogen (8) and a tail gas (9) from the separation of hydrogen;
it also comprises a step of combustion of a fuel stream selected from a fraction of the tail gas (9) from step g) and/or a fraction of the purified synthesis gas (5.2); mixed with a preheated air stream (15), which generates a flue gas stream (16), which gives off heat in the external heating of step a) and, after the pyrolysis step, gives off heat to at least one stream and/or fraction selected from: a stream of water (18) to generate steam (19) necessary for steps c) and f) and/or an air stream (14) to obtain the preheated air stream (15) and/or the fraction (5.1) prior to step f).

12. Method according to claim 11 **characterized in that** the pyrolysis step a) is carried out in a temperature range between 400°C and 650°C and the temperature of the cracking step b) is in a range between 800°C and 1300°C.

13. Method according to any one of the claims 11 to 12 **characterized in that** after the conversion step f), the hydrogen-enriched synthesis gas (7) is cooled by a stream of water which is subsequently heated to form the steam (19.1) for the cracking step and/or the water vapor (19.2) of the conversion step.

14. Method according to any one of the claims 11 to 13 **characterized in that** after conversion step f), the unreacted water vapor fraction of the hydrogen-enriched synthesis gas is condensed, and the condensed water is recycled together with the feed water (18).

15. A system to carry out the method of claims 1 to 10, **characterized in that** it comprises, at least:
a pyrolysis unit (20),
a cracking unit (21),
a purification unit (23),
a synthesis gas conversion unit (24),
a hydrogen separation unit (25),
a synthesis unit (26) for hydrocarbons or alcohols and
an unit for fractionation and/or purification of hydrocarbons or alcohols (27).
and wherein the cracking unit includes a cracking reactor and a unit for cooling raw synthesis gas.

16. A system to carry out the method of claims 11 to 14 comprising, at least:
a pyrolysis unit (20),
a cracking unit (21),
a purification unit (23),
a synthesis gas conversion unit (24),
a hydrogen separation unit (25) and
a section for recovering residual heat from the flue gas (28).
and wherein the cracking unit includes a cracking reactor and a unit for cooling raw synthesis gas.
